# EUROPEAN PATENT APPLICATION

(11) **EP 1 415 993 A1**
(43) Date of publication of application: **06.05.2004**
(21) Application number: 03023023.9
(22) Date of filing: 13.10.2003
(51) Int. Cl.: C07D 495/04, A61K 31/4365, A61P 7/02

(54) **Clopidogrel salts with alkyl-sulphuric acids**

(30) Priority: 21.10.2002 IT MI20022228
(71) Applicant: Dinamite Dipharma S.p.A. (in abbreviated form Dipharma S.p.A.), 33036 Mereto Di Tomba (UD) (IT)
(72) Inventor: Castaldi, Graziano, 28072 Briona (NO) (IT); Bologna, Alberto, 26013 Crema (CR) (IT); Magrone, Domenico, 20128 Milano (IT)
(74) Representative: Minoja, Fabrizio, Dr.

(57) **Abstract**

Clopidogrel salts with alkyl-sulphuric acids, having the following formula (I) 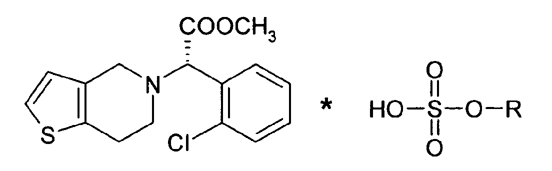 wherein R is a straight or branched C₁-C₁₀ alkyl group; preparation thereof and the industrial and therapeutical use thereof.

## Description

### DISCLOSURE

The present invention relates to novel, pharmaceutically acceptable clopidogrel salts with alkyl-sulphuric acids in the crystalline form, a process for the preparation thereof, and pharmaceutical compositions containing them.

(S)-2-(2-Chlorophenyl)-2-(4,5,6,7-tetrahydrothieno[3,2-c]-5-pyridyl)-acetic acid methyl ester is known from FR-A- 2 530 247 as a racemic mixture. EP-A-281459 discloses that the dextro-enantiomer form, known as clopidogrel, has biological activity as anti-platelet agent and is therefore used as a medicament for the prevention and treatment of the thrombotic disease. Furthermore, according to EP-A-281459, a number of clopidogrel salts, obtained with acids conventionally used in the pharmaceutical technique, precipitate in the amorphous form and/or are hygroscopic, which makes their use on industrial scale difficult.

Absorption of humidity represents a very serious problem in the pharmaceutical technology of powders. Humidity is in fact known to significantly affect the physical and chemical characteristics of medicaments and excipients and hence the resulting formulations. Absorption of humidity is therefore a key factor to be considered when dealing with preparation of pharmaceutical formulations, as well as packaging and storage of medicaments.

As a consequence, there is a need for novel clopidogrel salts. EP-A-281459 has partly solved the above mentioned problem by providing the sulfate salt of said compound.

The inventors of the present invention have surprisingly found novel clopidogrel salts having peculiar chemico-physical characteristics, which make their use particularly advantageous in pharmaceutical chemistry, pharmaceutical technique and therapy.

A first object of the present invention is therefore a novel salt of the dextro-enantiomer of (S)-2-(2-chlorophenyl)-2-(4,5,6,7-tetrahydrothieno[3,2-c]-5-pyridyl)acetic acid, i.e. clopidogrel, with an alkyl-sulfuric acid, having the following formula (I) 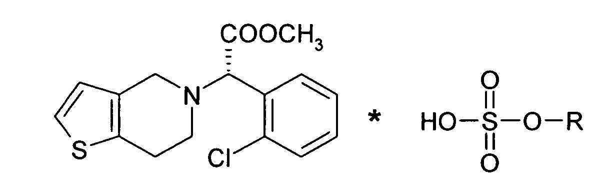 wherein R is a straight or branched C₁-C₁₀ alkyl group.

According to the invention, a C₁-C₁₀ alkyl group can be, for example, a straight or branched C₁-C₆ alkyl group, such as methyl, ethyl, propyl, isopropyl, butyl, sec-butyl, isobutyl, tert-butyl and pentyl, preferably a straight or branched C₁-C₄ alkyl group such as methyl, ethyl, propyl, isopropyl, butyl, sec-butyl, isobutyl or tert-butyl, most preferably isopropyl or sec-butyl.

Preferred compounds of formula (I) are the compounds in which R is a straight or branched C₁-C₆ alkyl group.

Specific examples of preferred compounds of formula (I) are:
clopidogrel-methyl sulfate; clopidogrel-ethyl sulfate; clopidogrel-propyl sulfate; clopidogrel-isopropyl sulfate; clopidogrel-butyl sulfate; clopidogrel-sec-butyl sulfate; clopidogrel-isobutyl sulfate and clopidogrel-tert-butyl sulfate.

Specific examples of more preferred compounds of formula (I) are:
clopidogrel-isopropyl sulfate and clopidogrel-sec-butyl sulfate.

A compound of formula (I) can be prepared with a process comprising:
a) reaction of clopidogrel with concentrated sulfuric acid in a C₁-C₁₀ alkanol; or
b) reaction of clopidogrel hemisulfate with a C₁-C₁₀ alkanol; or
c) salification of clopidogrel with an alkylsulfuric acid of formula R-O-S(O)₃H, wherein R is as defined above.

According to step a), a compound of formula (I), i.e. a salt of clopidogrel with a C₁-C₁₀ alkyl-sulfuric acid, can be prepared by adding concentrated sulfuric acid in stoichiometric amount to a solution of clopidogrel (base) in a C₁-C₁₀ alkanol, corresponding to the desired C₁-C₁₀ alkyl sulfate. In this reaction, commercially available concentrated sulfuric acid is used, preferably at a concentration of about 95-97%. The reaction is preferably carried out at reflux temperature, for a time ranging from about 2 to about 6 hours. After cooling, the reaction solution is allowed to stand to obtain the highly pure compound of formula (I) in the crystalline form. Alternatively, the compound of formula (I) can be obtained by evaporation of the (alcoholic) solvent and subsequent crystallization in a suitable solvent, for example acetone.

According to step b), a compound of formula (I) can be obtained by refluxing a suspension of clopidogrel hemisulfate in a C₁-C₁₀ alkanol corresponding to the desired C₁-C₁₀ alkyl sulfate. The reaction is preferably carried out at reflux temperature, for a time ranging from about 2 to about 7 hours. After cooling, the reaction solution is allowed to stand to obtain the highly pure compound of formula (I) in the crystalline form. Alternatively, the compound of formula (I) can be obtained by evaporation of the (alcoholic) solvent and subsequent crystallization in a suitable solvent, for example acetone.

According to step c), the salification of clopidogrel (base) with an alkylsulfuric acid of formula R-O-S(O)₃H, wherein R is as defined above, to obtain a compound of formula (I), can be carried out according to known methods, for example, by reacting clopidogrel (base) with a suitable C₁-C₁₀ alkylsulfuric acid, in a solvent which is preferably the corresponding C₁-C₁₀ alkanol.

The crystallization procedure, herein described and carried out for example as indicated in the experimental section, provides a highly pure compound of formula (I) in a homogeneously crystalline, non hygroscopic form, which is very stable in time and has physical and rheological characteristics which make its use in chemistry and pharmaceutical technique easy, in particular in case of filtration, drying, sieving, formulation procedures and the like. "Highly pure" herein means having purity approx. equal to or higher than 99%, preferably approx. equal to or higher than 99.5%.

A further object of the invention is a compound of formula (I) in the crystalline form, either in the anhydrous or hydrate form, for example as obtainable according to the preparation process herein described.

Particularly preferred is clopidogrel isopropyl sulfate in the crystalline form, in particular in the form according to the diffractogram reported in the Table in the Experimental Section.

The process of the invention is extremely advantageous in that it is not necessary that clopidogrel base and clopidogrel hemisulfate used as the starting products, respectively according to step a) or b), are in a highly pure form. In fact, according to the invention, said products can be used in the crude form, therefore they can contain impurities and by-products commonly deriving from industrial preparation processes.

Furthermore, if desired, a compound of the invention, i.e. a highly pure clopidogrel C₁-C₁₀ alkyl-sulfate salt, can subsequently be converted to clopidogrel base which can in turn be converted to clopidogrel hemisulfate. Said conversion processes can be carried out according to known methods. Clopidogrel base and clopidogrel hemisulfate are thus obtained in good yields and with high purity level so as to fulfil the requirements established by the regulations for the preparation of galenic preparations.

Therefore, the present invention also relates to a process for the purification of clopidogrel (base) and clopidogrel hemisulfate, wherein the clopidogrel C₁-C₁₀ alkylsulfate salt plays a key role.

Said purification process envisages three alternatives:
a) reaction of clopidogrel with concentrated sulfuric acid in a C₁-C₁₀ alkanol; or
b) reaction of clopidogrel hemisulfate with a C₁-C₁₀ alkanol; or
c) salification of clopidogrel with an alkylsulfuric acid of formula R-O-S(O)₃H, wherein R is as defined above;
followed by conversion of the resulting clopidogrel C₁-C₁₀ alkyl sulfate salt to clopidogrel (base) and, if desired, salification to clopidogrel hemisulfate.

The present invention also relates to highly pure clopidogrel (base) and clopidogrel hemisulfate, as obtainable according to the purification process of the invention.

The present invention also relates to a pharmaceutical composition comprising a compound of the invention as the active ingredient, together with pharmaceutically acceptable excipients and/or carriers.

A pharmaceutical composition according to the invention can be formulated with known methods.

The dosage of a compound of the invention in pharmaceutical formulations for humans is substantially that indicated in the French patent application n. 2 530 247. For example, a pharmaceutical composition for the oral administration can contain an amount from about 50 to about 100 mg of clopidogrel isopropyl sulfate per unitary dose.

A further object of the invention is the use of a compound of formula (I) as a medicament, in particular as anti-platelet agent.

A further object of the invention is the use of a compound of formula (I) for the preparation of a pharmaceutical composition for the treatment of a disease which can be treated with anti-platelet agents.

A further object of the invention is a method for the treatment of mammals, humans included, in the need of an anti-platelet agent, comprising administering to said mammal a therapeutically effective amount of a compound of formula (I).

The following examples further illustrate the invention.

### Example 1 - Clopidogrel isopropyl sulfate

A 1.5 1 reactor, equipped with mechanical stirrer and condenser, was loaded at room temperature with clopidogrel hemisulfate (50 g, 0.12 mol) having HPLC purity = 99.5%, and isopropanol (500 ml). The mass was refluxed under stirring; in these conditions the solid dissolved completely in 2 hours. After about 5 hours, the reaction mixture was cooled to room temperature: the product precipitated after approx. 3 hours. The solid was filtered after about 15 hours and dried under vacuum (200 mm Hg) at a temperature of 60°C for 24 hours. 48.8 g of clopidogrel isopropyl sulfate was obtained (yield = 88.8%); m.p. 167.1°C; HPLC purity >99.9%.

### Example 2 - Clopidogrel isopropyl sulfate

A 250 ml flask, equipped with magnetic stirrer and condenser, was loaded at room temperature with clopidogrel hemisulfate (20 g, 47.7 mmol) having HPLC purity = 97.9%, and isopropanol (100 ml). The mass was refluxed under stirring; in these conditions the solid dissolved completely in 2 hours. After about 6 hours the reaction mixture was cooled to room temperature: the product precipitated after approx. 3 hours. The solid was filtered after about 15 hours and dried under vacuum (200 mm Hg) at a temperature of 60°C for 24 hours. 19.33 g of clopidogrel isopropyl sulfate was obtained (yield = 88.0%); m.p. 167.3°C; HPLC purity =98.9%.

### Example 3 - Clopidogrel isopropyl sulfate

A 100 ml flask, equipped with magnetic stirrer and condenser, was loaded at room temperature with clopidogrel hemisulfate (5 g, 11.9 mmol) having HPLC purity = 97.9%, and isopropanol (40 ml). The mass was refluxed under stirring; in these conditions the solid dissolved completely in approx. 1 hour. After about 7 hours the reaction mixture was cooled to room temperature: the product precipitated after approx. 3 hours. After about 15 hours at room temperature, the solid was cooled to 0°C on an ice bath. After approx. 2 hours the solid was filtered and dried under vacuum (200 mm Hg) at a temperature of 60°C for 24 hours. 4.75 g of clopidogrel isopropyl sulfate was obtained (yield = 86.4%); m.p. 167.3°C; HPLC purity >99.9%.

According to the same procedure, the following compounds were prepared:
clopidogrel-methyl sulfate; clopidogrel-ethyl sulfate; clopidogrel-propyl sulfate; clopidogrel-butyl sulfate; clopidogrel-sec-butyl sulfate; clopidogrel-isobutyl sulfate and clopidogrel-tert-butyl sulfate.

### Example 4 - Clopidogrel isopropyl sulfate

A 250 ml flask, equipped with magnetic stirrer and condenser, was loaded at room temperature with clopidogrel free base (15.3 g, 47.6 mmol) having HPLC purity = 97.9%, and isopropanol (100 ml). The resulting solution was added dropwise with 4.86 g of 96% H₂SO₄. The mass was refluxed under stirring. After about 4 hours, the reaction mixture was cooled to room temperature: the product precipitated after approx. 3 hours. After about 15 hours the solid was filtered and dried under vacuum (200 mm Hg) at a temperature of 60°C for about 24 hours. 18.7 g of clopidogrel isopropyl sulfate was obtained (yield = 85.0%); m.p. 167.3°C; HPLC purity =98.9%.

According to the same procedure, the following compounds were prepared:
clopidogrel-methyl sulfate; clopidogrel-ethyl sulfate; clopidogrel-propyl sulfate; clopidogrel-butyl sulfate; clopidogrel-sec-butyl sulfate; clopidogrel-isobutyl sulfate and clopidogrel-tert-butyl sulfate.

### Analytical results of the tests carried out on clopidogrel isopropyl sulfate. X-ray diffraction

The analysis was carried out with a Siemens P4 diffractometer with monochromatic radiation Cu-Kα (λ = 1.54179A°) using the scanning technique θ/2θ. Unit cell parametres were determined using 49 reflexions in the range 6 ≤ 2θ ≤ 40°.

The structure was resolved with the direct method using the SIR97 program which detects the position of all the atoms except for the hydrogens. Hydrogen atoms were calculated according to theoretical models and the final refinement was carried out with SHELXL97 program. The diffractogram is reported in the following table:

| θ (°) | Intensity |
|---|---|
| 11.08 | 1116.75 |
| 12.64 | 208 |
| 13.4 | 602.25 |
| 14.12 | 375.5 |
| 15.44 | 150.5 |
| 17.72 | 117.75 |
| 18.36 | 377 |
| 18.88 | 530.75 |
| 20.64 | 288.25 |
| 22.04 | 334.5 |
| 22.2 | 112 |
| 22.24 | 121.25 |
| 22.96 | 761 |
| 23.2 | 189 |
| 23.28 | 220 |
| 24.24 | 387.75 |
| 25.6 | 103 |
| 25.96 | 199.75 |
| 27.52 | 103 |
| 28.16 | 146.75 |
| 28.76 | 171.75 |
| 28.92 | 107 |
| 29.04 | 185.25 |

### Melting point

The melting point of the crystalline form ranges from 167.1 to 167.3°C, as measured with a Buchi B-545 apparatus with starting temperature of 130°C and heating speed of 1.0°C/min to 230°C.

### Nuclear Magnetic Resonance (NMR)

¹H-NMR analysis was carried out on 20 mg of product dissolved in CD₃OD. The following results were obtained:

¹H-NMR (CD₃OD): 1.27 ppm, 6H, d; 3.25 ppm, m, 2H; 3.75 ppm, m, 2H; 3.83 ppm, s, 3H; 4.22 ppm, d, 1H; 4.40 ppm, d, 1H; 4.58 ppm, m, 1H; 5.82 ppm, s, 1H; 6.80 ppm, d, 1H; 7.38 ppm, d, 1H; 7.42-7.75 ppm, m, 4H.

¹³C-NMR analysis was carried out on 50 mg of product dissolved in CD₃OD. The following results were obtained:

¹³C-NMR (CD₃OD): 21.98 ppm, 22.13 ppm, 47.10 ppm, 50.89 ppm, 53.58 ppm, 66.18 ppm, 72.39 ppm, 124.99 ppm, 125.68 ppm, 126.88 ppm, 127.04 ppm, 128.73 ppm, 130.33 ppm, 131.14 ppm, 131.71 ppm, 132.93 ppm, 135.52 ppm, 167.41 ppm.

### Example 5 - Clopidogrel sec-butyl sulfate

A 250 ml flask, equipped with magnetic stirrer and condenser, was loaded at room temperature with clopidogrel hemisulfate (15 g, 35.8 mmol), having HPLC purity = 99.5%, and sec-butanol (150 ml). The mass was refluxed under stirring; in these conditions the solid dissolved completely in 2 hours. After about 2 hours, the reaction mixture was cooled to room temperature and concentrated under vacuum to 30 ml. The resulting precipitate was filtered and dried under vacuum (200 mm Hg) at a temperature of 60°C for 24 hours. 14.2 g of clopidogrel sec-butyl sulfate was obtained (yield = 83.5%); m.p. 131,4°C; HPLC purity >99.9%.

### Example 6 - Clopidogrel isopropyl sulfate

A 250 ml flask, equipped with magnetic stirrer and condenser, was loaded at room temperature with clopidogrel free base (10 g, 31.1 mmol) and isopropanol (50 ml). The resulting solution was thermostatized at 25°C and added dropwise with 4.36 g of iso-propanesulfuric acid, in 10 minutes. After 3 hours the product precipitated. After cooling to about 0°C for 5 hours, the solid was filtered and dried under vacuum (200 mm Hg) at a temperature of 60°C for 24 hours. 12.6 g of clopidogrel isopropyl sulfate was obtained (yield = 87.7%); m.p. 167.3°C.

### Example 7 - Purification of clopidogrel (base) and conversion to clopidogrel hemisulfate

A 250 ml flask, equipped with magnetic stirrer and condenser, was loaded at room temperature with an approx. 20% solution of clopidogrel (crude free base) (15.3 g, 47.6 mmol; HPLC purity = 96.3%) in isopropanol (61.2 g). The resulting solution was added dropwise with 4.86 g of 96% H₂SO₄ and stirred under reflux. After about 4 hours the reaction mixture was cooled to room temperature: the product (clopidogrel isopropyl sulfate) precipitated after approx. 3 hours. After about 15 hours, the solid was filtered (21.4 g, 46.4 mmol, yield = 97.4%), placed in a 500 ml flask, then suspended in 150 ml of water and 150 ml of toluene. The stirred mass was added with 4.0 g (47.6 mmol) of sodium bicarbonate in portions. After separation, the organic phase containing clopidogrel (base) was washed twice with 50 ml of water, and then evaporated under vacuum to a residue. 60 g of acetone was added, and the resulting solution was thermostatized at 20°C and added dropwise with 4.74 g of 96% sulfuric acid. The solution was kept under stirring: after approx. 2 hours a precipitate began to form. After 20 hours the suspension was filtered to obtain a solid, which was dried under vacuum in a static dryer at 60°C for 24 hours. The resulting product (clopidogrel hemisulfate) was obtained in high yield and purity.

## Claims

1. A clopidogrel salt having the following formula (I) 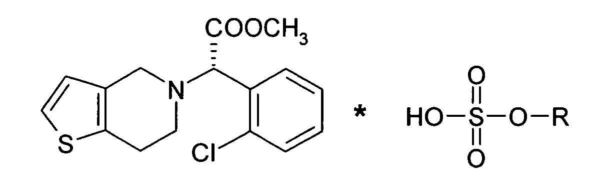 wherein R is a straight or branched C₁-C₁₀ alkyl group.

2. A clopidogrel salt as claimed in claim 1, wherein R is a straight or branched C₁-C₆ alkyl group.

3. A clopidogrel as claimed in claim 1, which is:
clopidogrel-methyl sulfate; clopidogrel-ethyl sulfate; clopidogrel-propyl sulfate; clopidogrel-isopropyl sulfate; clopidogrel-butyl sulfate; clopidogrel-sec-butyl sulfate; clopidogrel-isobutyl sulfate or clopidogrel-tert-butyl sulfate.

4. A pharmaceutical composition comprising as active ingredient a clopidogrel salt, as claimed in claim 1, and a pharmaceutically acceptable excipient and/or carrier.

5. A process for the purification of clopidogrel or clopidogrel hemisulfate, comprising respectively:
a) reaction of clopidogrel with concentrated sulfuric acid in a C₁-C₁₀ alkanol; or
b) reaction of clopidogrel hemisulfate with a C₁-C₁₀ alkanol; or
c) salification of clopidogrel with an alkylsulfuric acid of formula R-O-S(O)₃H, wherein R is as claimed in claim 1;
and conversion of the resulting clopidogrel C₁-C₁₀ alkyl sulfate salt to clopidogrel (base) and, if desired, salification to clopidogrel hemisulfate.

6. Highly pure clopidogrel and clopidogrel hemisulfate as obtainable according to the purification process as claimed in claim 5.

7. Crystalline form of a clopidogrel salt as claimed in claim 1.
